# EUROPÄISCHE PATENTANMELDUNG

(11) **EP 4 817 651 A1**
(43) Veröffentlichungstag der Anmeldung: **07.10.2026**
(21) Anmeldenummer: 25168583.0
(22) Anmeldetag: 04.04.2025
(51) Int. Cl.: A61M 5/14, A61M 5/142, A61M 5/168, A61M 39/10

(54) **FLUIDTRANSFERVORRICHTUNG UND SYSTEM ZUR VERSORGUNG EINES PATIENTEN MIT EINEM MEDIZINISCHEN FLUID SOWIE VERFAHREN**

(71) Anmelder: ConnCons GmbH, 01307 Dresden (DE)
(72) Erfinder: Bauer, Tony, 01156 Dresden (DE)
(74) Vertreter: Witte, Weller und Partner Patentanwälte mbB Stuttgart

(57) **Zusammenfassung**

Es wird vorgeschlagen eine Fluidtransfervorrichtung (40) zur Versorgung eines Patienten (2) mit einem medizinischen Fluid, insbesondere mit mindestens einem Medikament in flüssiger Form, bereitzustellen. Die Fluidtransfervorrichtung (40) weist ein erstes Anschlussstück (41) mit einem ersten Fluidkanal (42) zur Übertragung des Fluids und einer ersten Datenleitung (61) zur Übertragung eines Kommunikationssignals auf. Die Fluidtransfervorrichtung (40) weist ferner ein zweites Anschlussstück (43) mit einem zweiten Fluidkanal (44) zur Übertragung des Fluids und einer zweiten Datenleitung (62) zur Übertragung eines Kommunikationssignals auf. Die Fluidtransfervorrichtung (40) weist einen dritten Fluidkanal (45) zur Übertragung des Fluids zwischen dem ersten Fluidkanal (42) des ersten Anschlussstücks (41) und dem zweiten Fluidkanal (44) des zweiten Anschlussstücks (43) auf. Die Fluidtransfervorrichtung (40) weist ferner eine Elektronikeinrichtung (70) auf. Die Fluidtransfervorrichtung (40) ist dazu eingerichtet, in einem ersten Betriebsmodus betrieben zu werden, in welchem eine Kommunikationsverbindung zwischen der ersten Datenleitung (61) des ersten Anschlussstücks (41) und der Elektronikeinrichtung (70) bereitgestellt wird. Die Fluidtransfervorrichtung (40) ist ferner dazu eingerichtet, in einem zweiten Betriebsmodus betrieben zu werden, in welchem eine Kommunikationsverbindung zwischen der ersten Datenleitung (61) des ersten Anschlussstücks (41) und der zweiten Datenleitung (62) des zweiten Anschlussstücks (43) bereitgestellt wird. Ferner werden ein entsprechendes System (1) und Verfahren (100) vorgeschlagen.

## Beschreibung

### ANWENDUNGSGEBIET UND STAND DER TECHNIK

Die Erfindung betrifft eine Fluidtransfervorrichtung zur Versorgung eines Patienten mit einem medizinischen Fluid, insbesondere mit mindestens einem Medikament in flüssiger Form. Die vorliegende Erfindung betrifft ferner System zur Versorgung eines Patienten mit einem medizinischen Fluid sowie ein Verfahren. Allgemein betrifft die vorliegende Erfindung ein medizinisches Fluidleit- und - transfersystem sowie Komponenten, die in einem solchen System Verwendung finden können. Insbesondere betrifft die Erfindung eine Vorrichtung zur Übertragung eines Fluids mit gleichzeitiger Datenkommunikation.

Ein medizinisches Fluidleit- und -transfersystem ist ein System zur Führung von Fluiden im Zusammenhang mit der medizinischen Behandlung eines Menschen oder eines Tieres. Ein solches System kann beispielsweise ein Infusionssystem oder ein Transfusionssystem oder ein Dialysesystem oder ein System zum extrakorporalen Gasaustauch oder ein extrakorporales Kreislaufunterstützungssystem oder eine Herz-Lungen-Maschine oder ein Ernährungssystem oder ein Drainagesystem für Körperflüssigkeiten, wie Urin, Wundsekret oder Darminhalt oder ein Beatmungssystem sein. Unter medizinischen Fluiden im Sinne der Erfindung können alle im Rahmen von medizinischen Therapien verwendeten Flüssigkeiten verstanden werden, beispielsweise auch Nährlösungen und Blutprodukte.

Ein beispielhaftes Fluidleit- und -transfersystem ist in der EP 4 008 009 B1, einer früheren erteilten Patentschrift der vorliegenden Anmelderin ConnCons GmbH, beschrieben. Ferner sind in der WO 2023/198686 A1, einer weiteren früheren Anmeldung der vorliegenden Anmelderin ConnCons GmbH, eine Infusionspumpe sowie ein Verfahren zu deren Betrieb beschrieben.

Aus Hygienegründen kann es sich bei Fluidtransfervorrichtungen um Einwegartikel handeln.

### AUFGABE UND LÖSUNG

Aufgabe der Erfindung ist es, eine weiter verbesserte Fluidtransfervorrichtung bereitzustellen. Insbesondere wäre es wünschenswert eine kostengünstige Herstellung zu ermöglichen, da es sich, wie vorstehend erläutert, bei Fluidtransfervorrichtungen um Einwegartikel handeln kann.

Gemäß einem ersten Aspekt der Erfindung wird vorgeschlagene eine Fluidtransfervorrichtung, insbesondere zur Versorgung eines Patienten mit einem medizinischen Fluid, insbesondere mit mindestens einem Medikament in flüssiger Form, bereitzustellen.

Die Fluidtransfervorrichtung weist ein erstes Anschlussstück mit einem ersten Fluidkanal zur Übertragung des Fluids und einer ersten Datenleitung zur Übertragung eines Kommunikationssignals auf. Die Fluidtransfervorrichtung weist ferner ein zweites Anschlussstück mit einem zweiten Fluidkanal zur Übertragung des Fluids und einer zweiten Datenleitung zur Übertragung eines Kommunikationssignals auf. Die Fluidtransfervorrichtung weist einen dritten Fluidkanal zur Übertragung des Fluids zwischen dem ersten Fluidkanal des ersten Anschlussstücks und dem zweiten Fluidkanal des zweiten Anschlussstücks auf.

Das erste und/oder zweite Anschlussstück kann beispielsweise in Art eines Luer-Konnektors ausgebildet sein.

Die Fluidtransfervorrichtung weist ferner eine Elektronikeinrichtung auf. Die Fluidtransfervorrichtung ist dazu eingerichtet, in einem ersten Betriebsmodus betrieben zu werden, in welchem eine Kommunikationsverbindung zwischen der ersten Datenleitung des ersten Anschlussstücks und der Elektronikeinrichtung bereitgestellt wird. Die Fluidtransfervorrichtung ist ferner dazu eingerichtet, in einem zweiten Betriebsmodus betrieben zu werden, in welchem eine Kommunikationsverbindung zwischen der ersten Datenleitung des ersten Anschlussstücks und der zweiten Datenleitung des zweiten Anschlussstücks bereitgestellt wird.

Wie in den einleitend genannten früheren Anmeldungen der vorliegenden Anmelderin beschrieben, ist es vorteilhaft, eine kombinierte Fluid- und Datenverbindung bereitzustellen. Die Offenbarung der früheren Anmeldungen WO 2021/023639 A1 bzw. der daraus hervorgegangene EP4008009B1 sowie der WO 2023/198686 A1 werden ausdrücklich durch Bezugnahme mit aufgenommen. Im Rahmen der vorliegenden Offenbarung kann unter einem Anschlussstück insbesondere eine Kopplungseinrichtung am Ende einer Fluidleitung oder als Eingang oder Ausgang eines medizinischen Geräts, insbesondere einer Fluidtransfervorrichtung, verstanden werden, die mit einem korrespondierenden gegensätzlichen Gegen-Anschlussstück oder mit einem identischen Anschlussstück mechanischen gekoppelt werden kann, um die Anschlussstücke in die Lage für Fluidaustausch zu versetzen. Im Rahmen der vorliegenden Offenbarung kann ein Anschlussstück einen durch einen Leitungsabschnitt, insbesondere einer Datenleitung, umgebenen Fluidkanal zur umfänglich dichten Ankopplung in einer Steckrichtung an ein Gegen-Anschlussstück und zum nachfolgenden Austausch von Flüssigkeiten und/oder Gasen mit dem Gegen-Anschlussstück aufweisen. Das Anschlussstück kann eine Übertragungseinrichtung zur induktiven Datenübertragung mit dem Gegen-Anschlussstück aufweisen. Die Übertragungseinrichtung kann mit einer jeweiligen Datenleitung des Anschlussstücks verbunden sein. Die Übertragungseinrichtung kann mindestens eine planare Spule aufweisen, die den Fluidkanal umgebend ausgebildet ist. Alternativ oder zusätzlich kann die Übertragungseinrichtung eine den Fluidkanal umgebende, insbesondere schraubenförmige, Spule aufweisen.

Die Erfinder haben erkannt, dass es bei einem Fluidleit- und -transfersystem wünschenswert wäre, mit den das System bildenden Vorrichtungen zu kommunizieren und insbesondere eine Topologie des Systems bestimmen zu können. Hierdurch kann beispielsweise verifiziert werden, dass die richtigen Komponenten in der richtigen Reihenfolge bzw. an der richtigen Position angeschlossen wurden. Beispielsweise kann ein Vergleich von einer Ist-Topologie mit einer Soll-Topologie vorgenommen werden. Es ist denkbar, dass eine Hauptvorrichtung, wie beispielsweise eine Infusionspumpe oder ein Dialysegerät, einen Fluidtransfer erst dann freigibt, wenn eine korrekte Topologie des Fluidleit- und transfersystems bestimmt wurde.

Da es sich bei den Systemkomponenten zumindest teilweise um Einwegartikel handeln kann, wäre es wünschenswert, eine kostengünstige Herstellung zu ermöglichen. Ferner wäre es wünschenswert, in den Systemkomponenten den Einsatz kostengünstiger Elektronikeinrichtungen auf einfache Art und Weise zu ermöglichen.

Gemäß einem Aspekt der vorliegenden Erfindung wird daher gezielt für Fluidtransfervorrichtungen, welche einen dritten Fluidkanal zur Übertragung des Fluids zwischen dem ersten Fluidkanal des ersten Anschlussstücks und dem zweiten Fluidkanal des zweiten Anschlussstücks aufweisen, eine Ausgestaltung vorgeschlagen, welche einen einfachen und kostengünstigen Aufbau erlaubt und dennoch eine einfache, aber gezielte Kommunikation mit einer oder mehreren nachfolgenden Vorrichtungen ermöglicht. Die Erfinder haben ferner erkannt, dass im vorliegenden speziellen Anwendungsfall üblicherweise geringe Datenmengen von beispielsweise einigen Byte oder wenigen Kilobits ausreichend sein können. Beispielsweise kann eine Abfrage von Identifikationsnummern der am Fluidleit- und -transfersystem angeschlossenen Vorrichtungen ausreichen.

Statt also ein komplexes Netzwerk mit umfassenden und ggf. parallelen Kommunikationsmöglichkeiten zwischen mehreren Systemkomponenten bereitzustellen, wird eine Fluidtransfervorrichtung vorgeschlagen, welche dazu eingerichtet ist, in einem ersten und in einem zweiten Betriebsmodus betrieben zu werden. In dem ersten Betriebsmodus wird eine Kommunikationsverbindung zwischen der ersten Datenleitung des ersten Anschlussstücks und der Elektronikeinrichtung bereitgestellt. In diesem Betriebsmodus kann also gezielt mit der Elektronikeinrichtung der Fluidtransfervorrichtung kommuniziert werden. In dem zweiten Betriebsmodus wird hingegen eine Kommunikationsverbindung zwischen der ersten Datenleitung des ersten Anschlussstücks und der zweiten Datenleitung des zweiten Anschlussstücks bereitgestellt. Der zweite Betriebsmodus kann einen Bypass bereitstellen, in welchem ein über die erste Datenleitung des ersten Anschlussstücks empfangenes Kommunikationssignal einfach und insbesondere ohne weitere Signalverarbeitung an das zweite Anschlussstück - und damit an nachfolgende Elemente - eines Fluidleit- und -transfersystem weitergeleitet werden kann.

Ein weiterer Vorteil kann darin bestehen, dass Kollisionen in der Kommunikation auf einfache Art und Weise reduziert oder vermieden werden können. Hierdurch kann die Signalverarbeitung in Rahmen der Kommunikation vereinfacht werden. Dies kann den Einsatz kostengünstigerer Komponenten ermöglichen, was insbesondere für Einwegartikel vorteilhaft ist.

Ein weiterer Vorteil kann darin bestehen, dass ein energiesparender Aufbau ermöglicht wird. Die vorgeschlagene Fluidtransfervorrichtung und/oder weitere Elemente eines Fluidleit- und -transfersystems können dazu eingerichtet sein, über die Datenleitung nicht nur mit einem Kommunikationssignal, sondern auch mit Energie versorgt zu werden. Bei galvanisch entkoppelten Anschlusstücken kann hierbei drahtlose Energieübertragung bzw. WPT (wireless power transfer) eingesetzt werden. Die übertragbaren Leistungen bzw. Energiemengen pro Zeiteinheit sind hierbei begrenzt. In dem ersten Betriebsmodus kann die Energie gezielt der Elektronikeinrichtung der Fluidtransfervorrichtung zugeführt werden. In dem zweiten Betriebsmodus kann die Energie gezielt an das zweite Anschlussstück weitergeleitet werden.

Ein weiterer Vorteil kann darin bestehen, dass die vorgeschlagene Ausgestaltung den Einsatz von NFC-Elektronikeinrichtungen ermöglichen kann. Die Elektronikeinreichung kann einen NFC-Chip aufweisen. Ein NFC-Chip kann dazu eingerichtet sein, in Erwiderung auf ein empfangenes Kommunikationssignal ein Antwortsignal bereitzustellen. Das Antwortsignal kann beispielsweise eine ID oder Identifikationsnummern aufweisen. Alternativ oder zusätzlich kann eine begrenzte Datenmenge bereitgestellt werden. Diese kann für Anwendungen in medizinischen Fluidleit- und -transfersystemen jedoch ausreichen. Ein Nachteil bei NFC-Chips besteht darin, dass diese üblicherweise antworten, sobald eine Anfrage sie erreicht. Wenn mehrere NFC-Chips nahe beieinander angeordnet sind, kann es also vorkommen, dass mehrere der NFC-Chips antworten. Die Anordnung bzw. Topologie der NFC-Chips und ihrer zugehörigen Geräte wäre nicht mehr feststellbar. Mit der vorgeschlagenen Lösung ist es jedoch möglich, ein Kommunikationssignal (kabelgebunden) über das erste Anschlussstück zu erhalten. In dem ersten Betriebsmodus wird eine Kommunikationsverbindung zwischen der ersten Datenleitung des ersten Anschlussstücks und der Elektronikeinrichtung bereitgestellt. Das (NFC-)Kommunikationssignal wird der (NFC-)Elektronikeinrichtung der Fluidtransfervorrichtung zugeführt. In dem zweiten Betriebsmodus wird eine Kommunikationsverbindung zwischen der ersten Datenleitung des ersten Anschlussstücks und der zweiten Datenleitung des zweiten Anschlussstücks bereitgestellt. Das (NFC-)Kommunikationssignal wird also (kabelgebunden) an das zweite Anschlussstück weitergeleitet und kann gezielt (kabelgebunden) an nachfolgende Systemelemente weitergeleitet werden. Somit können kostengünstige NFC-Chips eingesetzt und dennoch gezielt angesprochen werden. Indem nacheinander verschiedene Systemelemente angesprochen werden, ist es auch möglich trotz Einsatz von kostengünstiger NFC-Elektronik eine Topologie des Fluidleit- und -transfersystems aufzulösen.

Ein weiterer Vorteil kann darin bestehen, dass die Sicherheit in einem Fluidleit- und transfersystem weiter verbessert werden kann. Indem gezielt entweder eine Kommunikationsverbindung mit der Elektronikeinrichtung der Fluidtransfervorrichtung oder dem zweiten Anschlussstück bereitgestellt wird, kann ein Risiko, dass die Elektronikeinrichtung der Fluidtransfervorrichtung von nachgelagerten Elementen kompromittiert wird, reduziert werden.

Es versteht sich, dass in dem dritten Fluidkanal ein Funktionselement angeordnet sein kann. Das Funktionselement kann dazu eingerichtet sein, das Fluid zu manipulieren. Beispielsweise kann ein Filter, ein Ventil, eine Luftfalle, ein Abscheider, eine Weiche, ein Kombinierer oder Umschalter zur Zuführung oder Abführung weiterer medizinischer Fluide oder dergleichen vorgesehen sein. Im Rahmen der vorliegenden Offenbarung kann unter einer Fluidtransfervorrichtung mit einem dritten Fluidkanal zur Übertragung des Fluids zwischen dem ersten Fluidkanal des ersten Anschlussstücks und dem zweiten Fluidkanal des zweiten Anschlussstücks auch eine Vorrichtung verstanden werden, bei welcher ein weiteres Anschlussstück mit einem weiteren Fluidkanal vorhanden ist und der erste Fluidkanal des ersten Anschlussstücks und/oder der zweite Fluidkanal des zweiten Anschlussstücks mit dem weiteren Fluidkanal des weiteren Anschlussstücks fluidisch koppelbar oder gekoppelt sind. Statt einer direkten Verbindung ist somit auch denkbar, dass jeweils das ersten Anschlussstück oder das zweite Anschlussstück oder beide fluidisch mit einem weiteren Anschlussstück verbunden sind. Beispielsweise können das erste und zweite Anschlussstück Fluideingänge sein, die einem weiteren Anschlussstück als gemeinsamen Fluidausgang zugeführt werden. Die Kommunikationsverbindung wird im zweiten Betriebsmodus dennoch zwischen dem ersten Anschlussstück und dem zweiten Anschlussstück bereitgestellt. Dies kann beispielsweise vorteilhaft sein, wenn an einen Kombinierer mehrere Zweige eines Fluidleit- und -transfersystems angeschlossen sind.

Gemäß einem weiteren Aspekt der vorliegenden Erfindung wird ein System, insbesondere zur Versorgung eines Patienten mit einem medizinischen Fluid, insbesondere mit mindestens einem Medikament in flüssiger Form, vorgeschlagen mit den folgenden Merkmalen: einer Hauptvorrichtung, insbesondere einer Infusionspumpe; einer ersten Fluidtransfervorrichtung wie im Rahmen der vorliegenden Offenbarung beschrieben; einer zweiten Fluidtransfervorrichtung wie im Rahmen der vorliegenden Offenbarung beschrieben; wobei ein erstes Anschlussstück der ersten Fluidtransfervorrichtung dazu eingerichtet ist, mit einem korrespondierenden Anschlussstück der Hauptvorrichtung gekoppelt zu werden; wobei ein erstes Anschlussstück der zweiten Fluidtransfervorrichtung dazu eingerichtet ist, mit einem korrespondierenden zweiten Anschlussstück der ersten Fluidtransfervorrichtung gekoppelt zu werden; wobei das System dazu eingerichtet ist, die erste Fluidtransfervorrichtung zunächst in einem ersten Betriebsmodus zu betreiben, in welchem eine Kommunikationsverbindung zwischen der ersten Datenleitung des ersten Anschlussstücks und der Elektronikeinrichtung bereitgestellt wird, um eine Kommunikation der Hauptvorrichtung mit der Elektronikeinheit der ersten Fluidtransfervorrichtung bereitzustellen; und wobei das System dazu eingerichtet ist, die erste Fluidtransfervorrichtung nachfolgend in einem zweiten Betriebsmodus zu betreiben, in welchem eine Kommunikationsverbindung zwischen der ersten Datenleitung des ersten Anschlussstücks und der zweiten Datenleitung des zweiten Anschlussstücks bereitgestellt wird; und die zweite Fluidtransfervorrichtung in einem ersten Betriebsmodus zu betreiben, in welchem eine Kommunikationsverbindung zwischen der ersten Datenleitung des ersten Anschlussstücks der zweiten Fluidtransfervorrichtung und der Elektronikeinrichtung der zweiten Fluidtransfervorrichtung bereitgestellt wird, um eine Kommunikation der Hauptvorrichtung mit der Elektronikeinheit der zweiten Fluidtransfervorrichtung bereitzustellen.

Gemäß einem weiteren Aspekt der vorliegenden Erfindung wird ein Verfahren, insbesondere zur Bereitstellung eines System zur Versorgung eines Patienten mit einem medizinischen Fluid, insbesondere mit mindestens einem Medikament in flüssiger Form, vorgeschlagen mit den folgenden Merkmalen: Bereitstellen einer Hauptvorrichtung, insbesondere einer Infusionspumpe; Bereitstellen einer ersten Fluidtransfervorrichtung wie im Rahmen der vorliegenden Offenbarung beschrieben; Bereitstellen einer zweiten Fluidtransfervorrichtung wie im Rahmen der vorliegenden Offenbarung beschrieben; Koppeln eines ersten Anschlussstücks der ersten Fluidtransfervorrichtung mit einem korrespondieren-den Anschlussstück der Hauptvorrichtung; Koppeln eines ersten Anschlussstücks der zweiten Fluidtransfervorrichtung mit einem korrespondierenden zweiten Anschlussstück der ersten Fluidtransfervorrichtung; Betreiben der ersten Fluidtransfervorrichtung zunächst in einem ersten Betriebsmodus, in welchem eine Kommunikationsverbindung zwischen der ersten Datenleitung des ersten Anschlussstücks und der Elektronikeinrichtung bereitgestellt wird; und Bereitstellen einer Kommunikation der Hauptvorrichtung mit der Elektronikeinheit der ersten Fluidtransfervorrichtung; Betreiben der ersten Fluidtransfervorrichtung nachfolgend in einem zweiten Betriebsmodus, in welchem eine Kommunikationsverbindung zwischen der ersten Datenleitung des ersten Anschlussstücks und der zweiten Datenleitung des zweiten Anschlussstücks bereitgestellt wird; und Betreiben der zweiten Fluidtransfervorrichtung in einem ersten Betriebsmodus, in welchem eine Kommunikationsverbindung zwischen der ersten Datenleitung des ersten Anschlussstücks der zweiten Fluidtransfervorrichtung und der Elektronikeinrichtung der zweiten Fluidtransfervorrichtung bereitgestellt wird; und Bereitstellen einer Kommunikation der Hauptvorrichtung mit der Elektronikeinheit der zweiten Fluidtransfervorrichtung.

Das erste und/oder zweite Anschlussstück kann ein Luer-Konnektor sein, insbesondere ein Luer-Konnektor gemäß der Normenfamilie ISO 80369. Sie dienen dem Anschluss an ein entsprechendes Gegenanschlussstück einer Schlauchleitung. Ein Vorteil dieser Ausgestaltung kann darin bestehen, dass eine zuverlässige Verbindung bereitgestellt werden kann.

Das erste und/oder zweite Anschlussstück kann zur kombinierten Übertragung des Fluids und zur induktiven Übertragung des Kommunikationssignals eingerichtet sein. Das erste und/oder zweite Anschlussstück kann eine Übertragungseinrichtung zur Datenübertragung und/oder zur Übertragung von elektrischer Energie aufweisen. Die Übertragungseinrichtung kann derart am Anschlussstück vorgesehen sein, dass sie mit einer korrespondierenden Übertragungseinrichtung an einem Gegen-Anschlussstück Daten austauschen oder Energie zuführen kann. Die Übertragungseinrichtung kann beispielsweise durch einen Abschnitt der jeweiligen Datenleitung gebildet werden. Zumindest ein Abschnitt der ersten Datenleitung kann den ersten Fluidkanal umgeben. Zumindest ein Abschnitt der zweiten Datenleitung kann den zweiten Fluidkanal umgeben. Hierbei kann der jeweilige Abschnitt der erste und/oder zweiten Datenleitung als Spule ausgebildet sein.

Die Übertragungseinrichtung eines jeweiligen Anschlussstückes kann demnach mit mindestens einer Spule zur induktiven Kopplung mit der Übertragungseinrichtung des Gegen-Anschlussstücks ausgebildet sein. Die induktive Übertragung sieht vor, dass unter Nutzung elektromagnetischer Induktion Signale zwischen zwei gekoppelten Anschlusstücken übertragen werden.

In vielen Anwendungsfällen kann die begrenzte Reichweite einer induktiven Datenübertragung und Energieübertragung als Nachteil angesehen werden. Im vorliegenden spezifischen Anwendungsfall einer Fluidtransfervorrichtung besteht jedoch genau darin ein Vorteil. Denn es erfolgt eine gezielte Datenübertragung und Energieübertragung zu einer über das Anschlussstück verbundenen Fluidtransfervorrichtung. Andere, nicht verbundene Vorrichtungen erhalten kein ausreichend starkes Signal, sodass keine unerwünschten Antwortsignale von anderen Vorrichtungen bereitgestellt werden. Somit kann eine auf ein Kommunikationssignal antwortende Fluidtransfervorrichtung auch als die tatsächlich angeschlossenen Fluidtransfervorrichtung identifiziert werden, welche sich in dem ersten Betriebsmodus befindet. Wenn sich die Fluidtransfervorrichtung in dem zweiten Betriebsmodus befindet, kann ein "Bypass" der Elektronikeinrichtung der Fluidtransfervorrichtung erfolgen. In diesem Fall kann eine auf ein Kommunikationssignal antwortende Fluidtransfervorrichtung als eine an das zweite Anschlussstück der (ersten) Fluidtransfervorrichtung angeschlossene weitere bzw. zweite Fluidtransfervorrichtung identifiziert werden, welche sich in dem ersten Betriebsmodus befindet. Auf diese Art und Weise kann eine Topologie eines Fluidnetzwerks mit einfachen und kostengünstigen Mitteln nach und nach aufgelöst werden.

Ein weiterer Vorteil eines ersten und/oder zweiten Anschlussstücks, das zur kombinierten Übertragung des Fluids und zur induktiven Übertragung des Kommunikationssignals eingerichtet ist, kann darin bestehen, dass eine galvanische Trennung von Elementen eines medizinischen Fluidleit- und -transfersystem bereitgestellt werden kann.

Die Fluidtransfervorrichtung kann dazu eingerichtet sein, in dem zweiten Betriebsmodus ein NFC-Signal drahtgebunden zwischen der ersten Datenleitung des ersten Anschlussstücks und der zweiten Datenleitung des zweiten Anschlussstücks weiterzuleiten. Im Rahmen der vorliegenden Offenbarung kann unter NFC insbesondere eine Kommunikation gemäß ISO/IEC 14443 verstanden werden. Die Near Field Communication (dt. Nahfeldkommunikation, abgekürzt NFC) ist ein auf der RFID-Technik basierender internationaler Übertragungsstandard zum kontaktlosen Austausch von Daten per elektromagnetischer Induktion mittels lose gekoppelter Spulen. Üblicherweise erfolgt eine vollständig drahtlose Übertragung über kurze Strecken von wenigen Zentimetern. Eine Datenübertragungsrate ist üblicherweise gering. Beispielsweise wird eine Datenübertragungsrate von maximal 424 kBit/s bereitgestellt. Im Rahmen der vorliegenden Erfindung wird jedoch vorgeschlagen, ein NFC-Signal drahtgebunden zwischen der ersten Datenleitung des ersten Anschlussstücks und der zweiten Datenleitung des zweiten Anschlussstücks weiterzuleiten. Ferner kann die Fluidtransfervorrichtung bzw. das System dazu eingerichtet sein NFC-Signale drahtgebunden mit einer kombinierten Fluid- und Datenleitung zwischen verschiedenen Elementen des Systems auszutauschen. Hierbei kann eine Fluidleitung eine integrierte Datenleitung, insbesondere mit einer einen Fluidkanal umgebenden differentiellen Twisted-Pair Datenleitung aufweisen. Indem die Fluidtransfervorrichtung kann dazu eingerichtet ist, in dem zweiten Betriebsmodus ein NFC-Signal drahtgebunden zwischen der ersten Datenleitung des ersten Anschlussstücks und der zweiten Datenleitung des zweiten Anschlussstücks weiterzuleiten, kann eine Weiterleitung an weitere Elemente ermöglicht werden. Insbesondere ist eine sogenannte "multi-hop" Verbindung über eine oder mehrere Fluidtransfervorrichtung möglich. Die Fluidtransfervorrichtung kann dazu eingerichtet sein, dass die Weiterleitung erst im zweiten Modus ermöglicht wird. Die Fluidtransfervorrichtung kann ferner an die bei NFC eingesetzten Trägerfrequenzen angepasst sein, beispielsweise zur Weiterleitung eines Signals mit einer Trägerfrequenz von 13,56 MHz.

Die Fluidtransfervorrichtung kann dazu eingerichtet sein, in dem ersten Betriebsmodus die zweite Datenleitung des zweiten Anschlussstücks von der ersten Datenleitung des ersten Anschlussstücks zu trennen. Hierbei kann eine galvanische Trennung bereitgestellt werden. Ein Vorteil dieser Ausgestaltung kann darin bestehen, dass die Weiterleitung des Kommunikationssignals unterbunden werden kann. Dies kann insbesondere bei der drahtlosen Energieversorgung vorteilhaft sein, da keine Energie unnötig an nachgelagerte Geräte weitergegeben wird. Ferner kann sich Sicherheit verbessert werden, da nachfolgende Geräte von der Kommunikation abgekoppelt sind. Es kann ferner vermieden werden, dass bei Einsatz von NFC-Kommunikation die NFC-Chips von nachgelagerten Geräten - welche ggf. nicht oder noch nicht einer bestimmten Position in der Topologie zugeordnet sind - abgefragt werden. Hierdurch kann das Problem von mehreren NFC-Chips (NFC-Tags) in einem Reader-Feld vermieden werden. Mit anderen Worten können in dem ersten Betriebsmoduls nachfolgende Knoten abgekoppelt sein und in dem zweiter Betriebsmodus ein nachfolgender Knoten angekoppelt sein.

Die Fluidtransfervorrichtung kann dazu eingerichtet sein, in dem zweiten Betriebsmodus die erste Datenleitung des ersten Anschlussstücks von der Elektronikeinrichtung zu trennen. Hierbei kann eine galvanische Trennung bereitgestellt werden. Ein Vorteil dieser Ausgestaltung kann darin bestehen, dass das Kommunikationssignal im zweiten Betriebsmodus ausschließlich weitergeleitet wird. Beispielsweise an das zweite Anschlussstück oder wenn weitere Anschlusstücke vorgesehen sind an eines der weiteren Anschlussstücke. Hierdurch kann insbesondere bei Energieversorgung über NFC, der Stromverbrauch weiter gesenkt werden. Mit anderen Worten kann die Fluidtransfervorrichtung dazu eingerichtet sein, dass nur in dem ersten Betriebsmoduls mit der Elektronikeinrichtung der Fluidtransfervorrichtung kommuniziert werden kann und die Elektronikeinrichtung in dem zweiten Betriebsmodus abgekoppelt ist. Beispielsweise kann damit verhindert werden, dass ein NFC-Chip der Elektronikeinrichtung auf ein Kommunikationssignal antwortet und gezielt eine Elektronikeinrichtung eines nachfolgenden Elements angesprochen werden.

In einer Weiterbildung kann die erste Datenleitung als differentielle erste Datenleitung ausgebildet sein. Die zweite Datenleitung kann als differentielle zweite Datenleitung ausgebildet sein. Die Fluidtransfervorrichtung kann einen Doppelpol-Umschalter (auch als DPDT-Schalter bzw. Double Pole Double Throw- Schalter bezeichnet), insbesondere einen Halbleiter DPDT-Schalter, zur Umschaltung zwischen dem ersten Betriebsmodus und dem zweiten Betriebsmodus aufweisen. Es versteht sich, dass bei mehreren Anschlusstücken auch ein Mehrkanal-Umschalter vorgesehen sein kann. Beispielsweise bei einer Fluidtransfervorrichtung, wie beispielsweise einem Dreiwegeventil, mit einem dritten Anschlussstück mit einem dritten Anschlussstück mit einem vierten Fluidkanal zur Übertragung des Fluids und einer dritten Datenleitung zur Übertragung eines Kommunikationssignals. Hierbei kann in dem ersten Betriebsmodus ein Kommunikationssignal mit einem entsprechenden Steuersignal an die Elektronikeinrichtung übermittelt werden und die Elektronikeinrichtung dazu eingerichtet sein, eine Umschaltung des Umschalters auf ein gewünschtes Anschlussstück zu veranlassen.

Die Fluidtransfervorrichtung kann dazu eingerichtet sein, in dem ersten Betriebsmodus zu starten. Die Fluidtransfervorrichtung kann dazu eingerichtet, selbsttätig, insbesondere nach einer vorgegebenen Zeitdauer, aus dem zweiten Betriebsmodus in den ersten Betriebsmodus zu wechseln. Ein Vorteil dieser Ausgestaltung besteht darin, dass zunächst eine Kommunikationsverbindung mit der Elektronikeinrichtung der Fluidtransfervorrichtung bereitgestellt wird. Somit kann eine Kommunikation mit einem definierten Element im Fluidleit- und -transfersystem aufgenommen werden. Durch den selbsttätigen Wechsel in den ersten Zustand, kann ferner sichergestellt werden, dass die Fluidtransfervorrichtung automatisch in denjenigen Zustand zurückfällt, in welchem nur die Elektronikeinheit der ersten Fluidtransfervorrichtung gekoppelt ist. Dies kann beispielsweise für eine zyklische Prüfung genutzt werden. Ferner können nachfolgende Geräte mit etwaigen Fehlfunktionen durch weiter "vorne" im Netzwerk angeordnete Fluidtransfervorrichtung auf einfache Art und Weise und ohne aufwändige Steuersignale von der Kommunikation ausgenommen werden.

Die Fluidtransfervorrichtung kann dazu eingerichtet sein, als Reaktion auf ein über die erste Datenleitung des ersten Anschlussstücks erhaltenes Steuersignal von dem ersten Betriebsmodus in den zweiten Betriebsmodus zu wechseln. Ein Vorteil kann in einer besonders einfachen, gezielten Steuerung bestehen. Vorzugsweise ist nur eine Elektronikeinrichtung eines einzelnen Elements des Fluidleit- und -transfersystems zur gleichen Zeit aktiv. Hierdurch kann der Energieverbrauch reduziert werde. Ferner kann vermieden werden, dass mehrere NFC-Chips zur gleichen Zeit antworten.

Die Fluidtransfervorrichtung kann dazu eingerichtet sein, induktiv über die Datenleitung des ersten Anschlussstücks mit Energie versorgt zu werden. Ein Vorteil dieser Ausgestaltung kann darin bestehen, dass ein kostengünstiger Aufbau ermöglicht wird. Dies ist insbesondere bei Einwegartikeln vorteilhaft. Die der Elektronikeinrichtung kann vorzugweise einen NFC-Chip aufweisen, welcher zur kombinierten Signal- und Energieübertragung eingerichtet ist. NFC-Chips sind kommerziell zu günstigen Preisen und in großen Stückzahlen verfügbar. Mit der vorgeschlagenen Lösung kann auf separate Einrichtungen und eine separate Verkabelung zur Energieversorgung verzichtet werden. Es versteht sich, dass optional ein Speicherelement zur Zwischenspeicherung der über die erste Datenleitung bereitgestellten Energie vorgesehen sein kann, beispielsweise ein Stützkondensator, insbesondere ein kostengünstiger Stützkondensator in Folientechnik.

Die Elektronikeinrichtung kann eine Eingangsschaltung zur Impedanzanpassung aufweisen. Die Eingangsschaltung kann dazu eingerichtet sein, abhängig von einer Position der Vorrichtung in einer Verschaltung von mehreren Fluidtransfervorrichtungen, insbesondere einer Position der Fluidtransfervorrichtung in einer Reihenschaltung von mehreren Fluidtransfervorrichtungen, eine mit der Position assoziierte vordefinierte Impedanzanpassung einzustellen. Die Eingangsschaltung kann zur dynamische Impedanzanpassung eingerichtet sein. Es kann ein optimiertes, adaptiertes Anpassnetzwerk vorgesehen sein. Die Eingangsschaltung kann umschaltbare Stufen bzw. Stages zur Impedanzanpassung aufweisen. Die Eingangsschaltung zur Impedanzanpassung kann einen Varaktor oder eine elektrisch steuerbare Kapazität aufweisen. Die Position der Fluidtransfervorrichtung in der Reihenschaltung von mehreren Fluidtransfervorrichtungen kann beispielsweise durch einen Master bzw. eine Hauptvorrichtung bestimmt werden. Je nachdem wie viele vorangehende Fluidtransfervorrichtungen von dem ersten in den zweiten Betriebsmodus umgeschaltet werden mussten, kann die Position bestimmt werden. Indem bei jeder Stufe mit jeder vorangehenden Fluidtransfervorrichtung keine 100%ige Energieübertragung erfolgt, kann abhängig von der Position in der Reihenschaltung eine andere Impedanzanpassung erforderlich sein. Allerdings kann die erforderliche Impedanzanpassung zu einem gewissen Grad deterministisch sein, so dass eine mit der Position assoziierte Impedanzanpassung eingestellt werden kann. Hierdurch kann die Energie- und oder Signalübertragung auf einfache und kostengünstige Art und Weise verbessert werden. Indem die Impedanzanpassung, insbesondere allein, basierend auf der Position erfolgt, kann auf eine aufwändige Impedanzmessung verzichtet werden. Damit kann eine kostengünstigere Elektronikeinrichtung eingesetzt werden.

Die Fluidtransfervorrichtung kann ein drittes Anschlussstück mit einem vierten Fluidkanal zur Übertragung des Fluids und einer dritten Datenleitung zur Übertragung eines Kommunikationssignals aufweisen. Die Fluidtransfervorrichtung kann einen fünften Fluidkanal zur Übertragung des Fluids zwischen dem ersten Fluidkanal des ersten Anschlussstücks und dem vierten Fluidkanal des dritten Anschlussstücks aufweisen. Es ist jedoch auch denkbar, dass eine Fluidverbindung zwischen dem zweiten Anschlussstück und dem dritten Anschlussstück bereitgestellt wird. Die Fluidtransfervorrichtung kann dazu eingerichtet sein, in einem dritten Betriebsmodus betrieben zu werden, in welchem in welchem eine Kommunikationsverbindung zwischen der ersten Datenleitung des ersten Anschlussstücks und der dritten Datenleitung des dritten Anschlussstücks bereitgestelltwird. Es versteht sich, dass auch entsprechende weitere Anschlusstücke und Betriebsmodi vorgesehen sein können. Eine beispielhafte Fluidtransfervorrichtung mit einem dritten Anschlussstück kann ein Dreiwegeventil sein. Eine beispielhafte Fluidtransfervorrichtung mit einer Mehrzahl von Anschlussstücken ist eine Filterbank.

In einer Weiterbildung des Systems, kann das System dazu eingerichtet sein, eine Netzwerktopologie der direkt oder indirekt an die Hauptvorrichtung angeschlossenen Fluidtransfervorrichtungen zu identifizieren durch sequenzielles Umschalten der ersten und zweiten Betriebsmodi der kaskadiert an die Hauptvorrichtung angeschlossenen Fluidtransfervorrichtungen. Hierbei kann die Hauptvorrichtung zunächst mit der ersten (direkt) an die Hauptvorrichtung angeschlossenen Fluidtransfervorrichtung kommunizieren. Wenn sich die erste Fluidtransfervorrichtung im ersten Betriebsmodus befindet, erhält die Hauptvorrichtung ein Antwortsignal der ersten Fluidtransfervorrichtung. Die Position der ersten Fluidtransfervorrichtung kann als direkt an die Hauptvorrichtung angeschlossen bestimmt werden. Durch ein Steuersignal von der Hauptvorrichtung kann die Fluidtransfervorrichtung von dem ersten Betriebsmodus in den zweiten Betriebsmodus versetzt werden. Ein Kommunikationssignal der Hauptvorrichtung wird demnach an eine an das zweite Anschlussstück der ersten Fluidtransfervorrichtung angeschlossene weitere Systemkomponente, wie beispielsweise eine zweite Fluidtransfervorrichtung weitergeleitet. Wenn sich die zweite Fluidtransfervorrichtung im ersten Betriebsmodus befindet, erhält die Hauptvorrichtung, weitergeleitet über die erste Fluidtransfervorrichtung, ein Antwortsignal der zweiten Fluidtransfervorrichtung. Die Position der zweiten Fluidtransfervorrichtung kann als an das zweite Anschlussstück der ersten Fluidtransfervorrichtung bestimmt werden. Indem die Position der ersten Fluidtransfervorrichtung im Netzwerk bekannt ist, dass somit die zweite Fluidtransfervorrichtung auf einfach und kostengünstige Art und Weise zur Topologie hinzugefügt werden. Es versteht sich, dass auch die Position weiterer Systemelemente wie beispielsweise von einem Endpunkt eines Arms des Systems, beispielsweise von einem Medikamentenbeutel insbesondere mit Elektronikeinrichtung, bestimmt werden können.

Das hierin beschriebene Vorgehen kann auch als "Sequential CallAnd Bypass", kurz SeCAB, bezeichnet werden. Die Systemkomponenten können mittels der sequenziell jeweils in den ersten Betriebsmodus versetzt und abgefragt werden und nachfolgen im zweiten Betriebsmodus per Bypass übergangen werden. Es wird somit eine weitere verbesserte Fluidtransfervorrichtung bereitgestellt, welche eine kostengünstige Herstellung ermöglicht und insbesondere auch als Einwegartikel ausgebildet sein kann.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
- Fig. 1: zeigt eine schematische Darstellung eines Systems zur Versorgung eines Patienten mit einem medizinischen Fluid.
- Fig. 2: zeigt eine schematische Darstellung einer direkten Verbindung einer Hauptvorrichtung mit einer weiteren Komponente eines Fluidsystems;
- Fig. 3: zeigt eine schematische Darstellung einer indirekten Verbindung einer Hauptvorrichtung mit einer oder mehreren weiteren Komponenten des Fluidsystems über eine Fluidtransfervorrichtung;
- Fig. 4: zeigt eine beispielhafte Ausgestaltung einer Fluidtransfervorrichtung;
- Fig. 5: zeigt eine weitere beispielhafte Ausgestaltung einer Fluidtransfervorrichtung;
- Fig. 6: zeigt eine weitere beispielhafte Ausgestaltung einer Fluidtransfervorrichtung;
- Fig. 7: zeigt beispielhafte Leistungsdiagramme;
- Fig. 8: zeigt eine schematische Darstellung mit Identifikation von Komponenten eines Fluidsystems; und
- Fig. 9: zeigt ein Flussdiagramm eines Verfahrens.

### DETAILLIERTE BESCHREIBUNG DER AUSFÜHRUNGSBEISPIELE

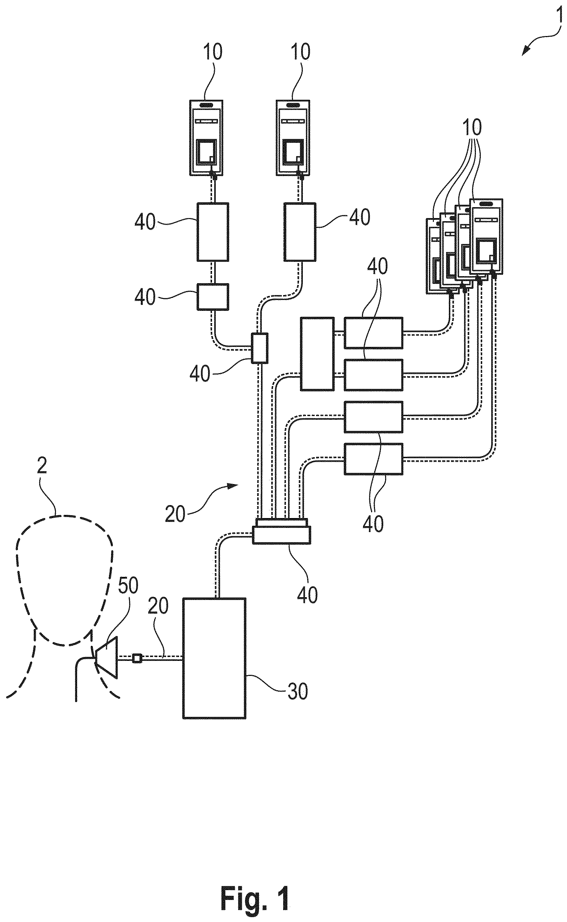

Fig. 1 zeigt eine schematische Darstellung eines Fluidleit- und -transfersystems als ein beispielhaftes System 1 zur Versorgung eines Patienten 2 mit einem medizinischen Fluid, insbesondere mit einem Medikament in flüssiger Form.

In dem gezeigten Beispiel kann das System 1 eine Anzahl von Medikationsbehältern 10 aufweisen, beispielsweise in Form von formflexiblen Beuteln. Im Rahmen der vorliegenden Offenbarung wird unter einem Medikationsbehälter ein Behälter für ein medizinisches Fluid verstanden. Die Medikationsbehälter 10 sind über Fluidleitungen 20 mit einer Hauptvorrichtung 30 verbunden. Bei der Hauptvorrichtung30 kann es sich beispielsweise um eine Infusionspumpe handeln. Die Hauptvorrichtung kann im vorliegenden Beispiel auch als Master bzw. Master Unit bezeichnet werden.

Das System 1 weist ferner eine oder mehrere Fluidtransfervorrichtungen 40 auf. Eine Fluidtransfervorrichtung kann beispielsweise einen Filter, ein Ventil, eine Luftfalle, einen Abscheider, eine Weiche, einen Kombinierer oder Umschalter zur Zuführung oder Abführung weiterer medizinischer Fluide oder dergleichen vorgesehen aufweisen. Beispielsweise kann eine Fluidtransfervorrichtung mit einer Tropfkammer, einem Rückschlagventil oder auch mit einem Dreiwegehahn oder Y-Stück vorgesehen sein. Die Fluidtransvorrichtungen und/oder Medikationsbehälter können jeweils auch als Slave bzw. Slave Unit bezeichnet werden.

Ferner kann ein Zugang 50 zur Übergabe des medizinischen Fluids an den Patienten 2 vorgesehen sein, beispielsweise eine Infusionsnadel, ein Port oder eine Viggo bzw. eine Venenverweilkanüle, die als intravenöser Zugang für die Verabreichung von Medikamenten, Elektrolytlösungen oder Flüssigkeit dienen kann.

Die Datenübertragung zwischen den Systemkomponenten erfolgt über Datenleitungen 21, die in die Fluidleitungen 20 integriert sind. Beispielhafte Fluidleitungen mit integrierten Datenleitungen sind in den eingangs genannten früheren Anmeldungen WO 2021/023639 A1 bzw. der daraus hervorgegangene EP4008009B1 sowie der WO 2023/198686 A1 beschrieben, deren diesbezügliche Offenbarungen ausdrücklich mit aufgenommen werden.

Wie in Fig. 1 dargestellt, kann ein System 1 zur Versorgung eines Patienten 2 mit einem medizinischen Fluid je nach Anwendungsfall eine komplexe Topologie mit einer Vielzahl von Komponenten bzw. Systemelementen 10, 20, 30 ,40, 50 aufweisen. Wie eingangs beschreiben wäre es wünschenswert, mit den das System bildenden Vorrichtungen zu kommunizieren und insbesondere eine Topologie des Systems bestimmen zu können. Hierdurch kann beispielsweise verifiziert werden, dass die richtigen Komponenten in der richtigen Reihenfolge bzw. an der richtigen Position angeschlossen wurden. Beispielsweise kann ein Vergleich von einer Ist-Topologie mit einer Soll-Topologie vorgenommen werden. Es ist denkbar, dass eine Hauptvorrichtung30, wie beispiels-weise eine Infusionspumpe oder ein Dialysegerät, einen Fluidtransfer erst dann freigibt, wenn eine korrekte Topologie des Fluidleit- und transfersystems bestimmt wurde. Da es sich um Einwegartikel handeln kann, wäre es wünschenswert eine kostengünstige Herstellung zu ermöglichen.

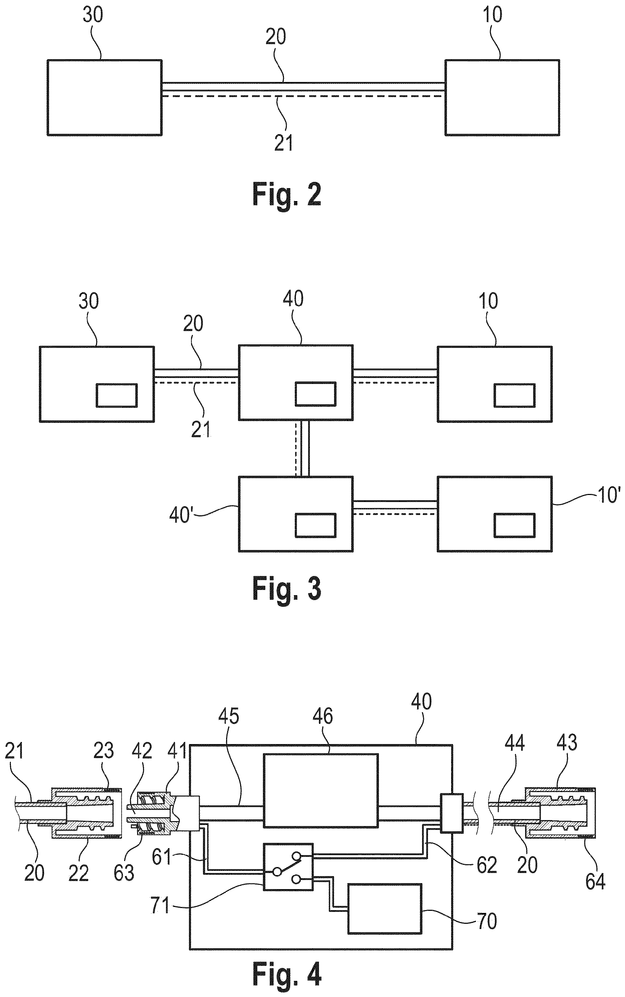

Fig. 2 zeigt eine schematische Darstellung einer direkten Verbindung einer Hauptvorrichtung 30 mit einer weiteren Komponente eines Fluidsystems, hier mit einem Medikationsbehälter 10. Die Hauptvorrichtung30 ist über eine Fluidleitung 20 mit integrierter Datenleitung 21 mit dem Medikationsbehälter 10 verbunden.

Fig. 3 zeigt eine schematische Darstellung einer indirekten Verbindung einer Hauptvorrichtung 30 mit einer oder mehreren weiteren Komponenten des Fluidsystems, hier mit einem ersten Medikationsbehälter 10 und einem zweiten Medikationsbehälter 10', über eine erste Fluidtransfervorrichtung 40 und eine optionale zweite Fluidtransfervorrichtung 40'. Die erste Fluidtransfervorrichtung 40 kann demnach einen weiteren Anschluss aufweisen, an welchen eine weitere Komponente des Fluidsystems wie beispielsweise ein zweiter Medikationsbehälter 10' oder eine zweite Fluidtransfervorrichtung 40' angeschlossen sein kann.

Fig. 4 zeigt eine beispielhafte Ausgestaltung einer Fluidtransfervorrichtung 40. Die Fluidtransfervorrichtung 40 weist ein erstes Anschlussstück 41 mit einem ersten Fluidkanal 42 zur Übertragung des Fluids und einer ersten Datenleitung 61 zur Übertragung eines Kommunikationssignals auf. Die Fluidtransfervorrichtung weist ferner ein zweites Anschlussstück 43 mit einem zweiten Fluidkanal 44 zur Übertragung des Fluids und eine zweiten Datenleitung 62 zur Übertragung eines Kommunikationssignals auf. Die Fluidtransfervorrichtung weist einen dritten Fluidkanal 45 zur Übertragung des Fluids zwischen dem ersten Fluidkanal 42 des ersten Anschlussstücks 41 und dem zweiten Fluidkanal 44 des zweiten Anschlussstücks 43 auf. Die Fluidtransfervorrichtung weist eine Elektronikeinrichtung 70 auf. Die Fluidtransfervorrichtung 40 ist dazu eingerichtet, in einem ersten Betriebsmodus betrieben zu werden, in welchem eine Kommunikationsverbindung zwischen der ersten Datenleitung 61 des ersten Anschlussstücks 41 und der Elektronikeinrichtung 70 bereitgestellt wird. Die Fluidtransfervorrichtung 40 ist ferner dazu eingerichtet, in einem zweiten Betriebsmodus betrieben zu werden, in welchem eine Kommunikationsverbindung zwischen der ersten Datenleitung 61 des ersten Anschlussstücks 41 und der zweiten Datenleitung 44 des zweiten Anschlussstücks 43 bereitgestellt wird.

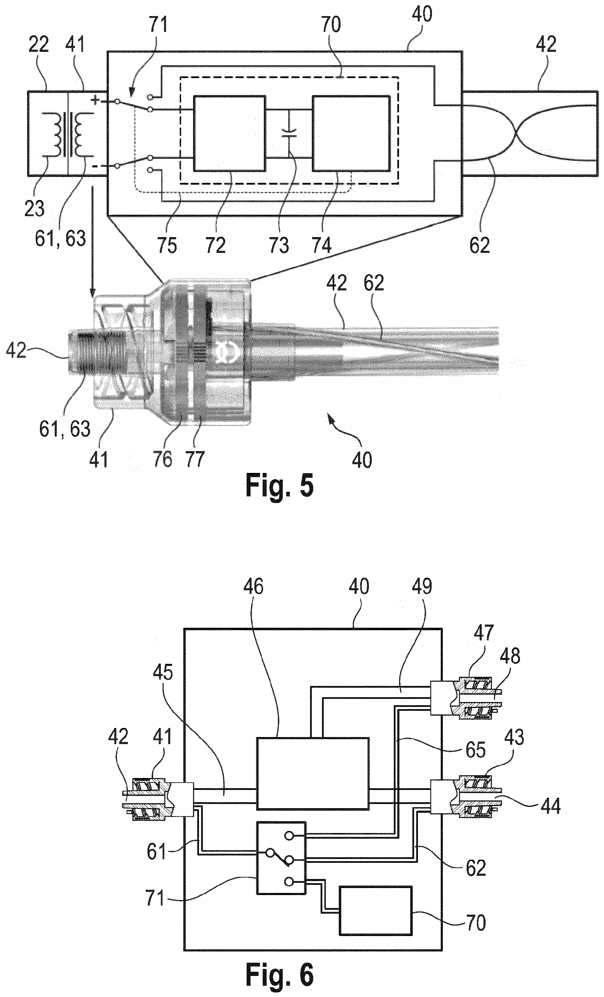

In dem dritten Fluidkanal 45 zur Übertragung des Fluids zwischen dem ersten Fluidkanal 42 des ersten Anschlussstücks 41 und dem zweiten Fluidkanal 44 des zweiten Anschlussstücks 43 kann optional ein Funktionselement 46 angeordnet sein. Beispielsweise kann ein Filter, ein Ventil, eine Luftfalle, ein Abscheider, eine Weiche, ein Kombinierer oder Umschalter zur Zuführung oder Abführung weiterer medizinischer Fluide (siehe auch Ausführungsbeispiel in Fig. 6) oder dergleichen vorgesehen sein.

Wie in Fig. 4 schematisch dargestellt, kann das erste Anschlussstück 41 und/oder das zweite Anschlussstück 43 als Luer-Konnektor ausgebildet sein. Hierbei ist in Fig. 4 auf der linken Seite ein Abschnitt einer Fluidleitung 20 mit einer integrierten Datenleitung 21 gezeigt. Diese kann beispiels-weise von einer Hauptvorrichtung 30 kommen oder auch von einer vorgelagerten Komponente des Fluidsystems. Beispielweise kann es sich bei der in Fig. 4 dargestellten Fluidtransfervorrichtung 40 um die in Fig. 3 mit Bezugszeichen 40' bezeichnete Fluidtransfervorrichtung handeln, mithin um eine zweite Fluidtransfervorrichtung, welche indirekt über eine vorgelagerte erste Fluidtransfervorrichtung 40 an eine Hauptvorrichtung angeschlossen ist.

Das Anschlussstück 41 kann eine mit der Datenleitung 61 gekoppelte Spule 63 zur induktiven Daten- und/oder Energieübertragung aufweisen. Entsprechend kann ein Anschlussstück 22 der Fluidleitung 20 eine korrespondierende Spule 23 zur induktiven Daten- und/oder Energieübertragung aufweisen. Das zweite Anschlussstück 43 kann ebenfalls eine mit der zweiten Datenleitung 62 gekoppelte Spule 64 zur induktiven Daten- und/oder Energieübertragung aufweisen. Bei der Spule 63 kann es sich um einen Abschnitt der ersten Datenleitung 61 handeln, welcher den ersten Fluidkanal 62 zumindest abschnittsweise umgibt. Bei der Spule 64 kann es sich um einen Abschnitt der zweiten Datenleitung 62 handeln, welcher den zweiten Fluidkanal 44 zumindest abschnittsweise umgibt. Allgemeiner kann das erste und/oder zweite Anschlussstück 41, 42 zur kombinierten Übertragung des Fluids und zur induktiven Übertragung des Kommunikationssignals eingerichtet sein.

Wie in Fig. 4 schematisch beim zweiten Anschlussstück dargestellt, kann die Fluidtransfervorrichtung zumindest einen Abschnitt einer Fluidleitung 20 aufweisen. Die jeweiligen Anschlussstücke können jedoch auch, wie beispielsweise in Fig. 6 dargestellt, jeweils an der Fluidtransfervorrichtung 40 angeordnet sein.

Die Elektronikeinrichtung 70 kann einen NFC-Chip aufweisen. Es ist auch möglich, dass die Elektronikeinrichtung als NFC-Chip ausgebildet ist. Statt allerdings mit einer Antenne gekoppelt zu werden, kann in dem ersten Betriebsmodus eine kabelgebundene Kommunikationsverbindung mit der ersten Datenleitung 61 des ersten Anschlussstücks 41 bereitgestellt werden.

Die Fluidtransfervorrichtung 40 ist ferner dazu eingerichtet in einem zweiten Betriebsmodus betrieben zu werden, in welchem eine Kommunikationsverbindung zwischen der ersten Datenleitung 61 des ersten Anschlussstücks 41 und der zweiten Datenleitung 62 des zweiten Anschlussstücks 42 bereitgestellt wird. Insbesondere kann die Fluidtransfervorrichtung 40 dazu eingerichtet sein, in dem zweiten Betriebsmodus ein NFC-Signal drahtgebunden zwischen der ersten Datenleitung 61 des ersten Anschlussstücks 41 und der zweiten Datenleitung 62 des zweiten Anschlussstücks 42 weiterzuleiten.

In dem in Fig. 4 gezeigten Beispiel ist hierfür ein Umschalter 71 vorgesehen. Der Umschalter 71 der beispielhaften Fluidtransfervorrichtung 40 ist dazu eingerichtet, in dem ersten Betriebsmodus die zweite Datenleitung 62 des zweiten Anschlussstücks 42 von der ersten Datenleitung 61 des ersten Anschlussstücks 41 zu trennen. Der Umschalter 71 der beispielhaften Fluidtransfervorrichtung 40 ist dazu eingerichtet, in dem zweiten Betriebsmodus die erste 61 Datenleitung des ersten Anschlussstücks 41 von der Elektronikeinrichtung 70 zu trennen. Fig. 4 zeigt ein vereinfachtes, schematisches Schaltersymbol, es sind jedoch verschiedene Ausgestaltungen des Umschalters denkbar. Der Umschalter kann auch mit der Elektronikeinrichtung ko-integriert sein. In diesem Fall ist es ausreichend, wenn in dem zweiten Betriebsmodus zumindest ein Teil der Funktionseinheiten der Elektronikeinrichtung abgekoppelt wird.

Fig. 5 zeigt ein beispielhaftes Blockdiagramm, insbesondere von elektrischen Komponenten, einer weiteren Ausgestaltung einer Fluidtransfervorrichtung 40. Im unteren Bereich von Fig. 5 ist ferner eine Abbildung eines prototypischen Aufbaus gezeigt.

Die erste und zweite Datenleitung 61, 62 können jeweils als differentielle Datenleitungen ausgebildet sein. Die Fluidtransfervorrichtung 40 kann einen Doppelpol-Umschalter 71, insbesondere einen Halbleiter DPDT-Schalter, zur Umschaltung zwischen dem ersten Betriebsmodus und dem zweiten Betriebsmodus aufweisen. In dem ersten Betriebsmodus ist die erste differentielle Datenleitung 61 durch den Umschalter 71 mit der Elektronikeinrichtung 70 gekoppelt. In dem zweiten Betriebsmodus kann die Elektronikeinrichtung 70 hingegen abgekoppelt und übergangen werden. Es wird ein "Bypass" bereitgestellt. Hierdurch stellt die Elektronikeinrichtung insbesondere keine Last dar. Das Kommunikationssignal kann in dem zweiten Betriebsmodus weitergeleitet werden, ob dass die Elektronikeinrichtung 70 der Fluidtransfervorrichtung 40 die Impedanz (nennenswert) beeinflusst.

Die Elektronikeinrichtung 70 kann eine Eingangsschaltung 71 aufweisen. Die Eingangsschaltung 71 kann dazu eingerichtet sein, dass über das erste Anschlussstück erhaltene (differentielle) Kommunikationssignal aufzubereiten. Beispielsweise kann die Schaltung dazu eingerichtet sein, Energie aus dem Kommunikationssignal zu ernten. Die Fluidtransfervorrichtung 40 ist dazu eingerichtet, induktiv über die Datenleitung 61 des ersten Anschlussstücks 41 mit Energie versorgt zu werden. Hierfür kann beispielsweise ein Gleichrichter, wie beispielsweise ein Brückengleichrichter vorgesehen sein. Ferner kann ein Energiespeicher 72 vorgesehen sein. In Fig. 5 ist der Energiespeicher 72 beispielhaft als Kondensator dargestellt, jedoch nicht hierauf beschränkt.

Ferner kann die Elektronikeinrichtung 70 Funktionselektronik 73 bzw. sogenannte Slave Elektronik aufweisen. Hierbei kann es sich um einen Mikrochip oder Mikroprozessor mit integriertem Speicher handeln. Die Slave-Elektronik ist zur Kommunikation mit der Hauptvorrichtung 30 eingerichtet. Hierbei kann in dem ersten Betriebsmodus ein Kommunikationssignal mit einem entsprechenden Steuersignal an die Elektronikeinrichtung 70 übermittelt werden und die Elektronikeinrichtung 70 dazu eingerichtet sein, eine Umschaltung des Umschalters 71 auf ein gewünschtes Anschlussstück, hier auf das zweite Anschlussstück 42 zu veranlassen. Dies kann beispielsweise über eine Steuerleitung 75 von der Elektronikeinrichtung 70 zum Umschalter 71 erfolgen.

Wie eingangs beschreiben ist die Fluidtransfervorrichtung 40 vorzugweise dazu eingerichtet, in dem ersten Betriebsmodus zu starten; und ist dazu eingerichtet, selbsttätig, insbesondere nach einer vorgegebenen Zeitdauer, aus dem zweiten Betriebsmodus in den ersten Betriebsmodus zu wechseln. Die Fluidtransfervorrichtung 40 ist vorzugweise dazu eingerichtet, als Reaktion auf ein über die erste Datenleitung 61 des ersten Anschlussstücks 41 erhaltenes Steuersignal von dem ersten Betriebsmodus in den zweiten Betriebsmodus zu wechseln.

Hierdurch kann auch die Betriebssicherheit verbessert werden. Sollten beispielsweise sich Vorrichtungen im Fluidnetzwerks befinden, die fehlerhaft agieren - durch Defekt oder durch bewusste Modifikation eines Angreifers - kann eine solche defekte oder kompromittierte Vorrichtung eben keine umfassende Verbindung zwischen den einzelnen Komponenten oder zu einer Vielzahl von Komponenten herstellen, die ausgenutzt werden könnte. In einem beispielhaften Szenario könnte ein Dreiwege-Hahn von einer Infusionspumpe als Hauptvorrichtung mittels Steuersignal, z.B. mittels NFC Command, umgeschaltet werden. Eine Tropfenkammer wird durch einen Angreifer softwaretechnisch modifiziert und ins System eingeschalten. Nun könnte diese Komponente die Kommunikation zwischen Hauptvorrichtung und Dreiwege-Hahn so stören, dass es zu fehlerhafter Stellung des Hahns und somit zu Gefahren kommt. Da gemäß einem Aspekt der der vorgeschlagenen Lösung immer nur die Hauptvorrichtung als sogenannter Ground-Truth Moderator mit einem einzelnen Teilnehmer spricht, ist ein Angriff durch in Masse hergestellte und bereitgestellte Verbrauchsmittel, wie beispielsweise Filter, Hähne, Tropfenkammern, nicht mehr ohne weiteres möglich. Anders gesagt können die eigentlichen Aktoren und Sensoren im Fluidnetzwerks sind durch den Moderator bzw. die Hauptvorrichtung voneinander isoliert. Somit gibt es eine Vulnerabilität weniger.

Fig. 5 zeigt im unteren Bereich ferner einen beispielhaften Aufbau einer Fluidtransfervorrichtung 40. Das erste Anschlussstück 41 ist als Luer-Konnektor ausgebildet. Die Elektronikeinrichtung 70 kann ebenfalls in das erste Anschlussstück 41 integriert sein. Vorzugweise kann die Elektronikeinrichtung 70 auf einer oder mehreren quer zum ersten Fluidkanal 42 angeordneten Platinen 76, 77 angeordnet sein. Die eine oder mehreren Platinen 76, 77 können den ersten Fluidkanal 42 zumindest teilweise, insbesondere vollständig, umgeben. Ein Energiespeicher kann optional zwischen zwei entsprechenden Platinen 76, 77 der Elektronikeinrichtung angeordnet sein.

Fig. 6 zeigt eine weitere beispielhafte Ausgestaltung einer Fluidtransfervorrichtung 40. Die Fluidtransfervorrichtung 40 kann ausgebildet sein, wie im Rahmen der vorliegenden Offenbarung beschrieben. Zur Vermeidung von Wiederholungen sollen, insbesondere die weiteren Merkmale im Vergleich zu Fig. 4 hervorgehoben werden.

Die in Fig. 6 gezeigte die Fluidtransfervorrichtung 40 weist ein drittes Anschlussstück 47 mit einem vierten Fluidkanal 48 zur Übertragung des Fluids und eine dritten Datenleitung 65 zur Übertragung eines Kommunikationssignals auf. Die Fluidtransfervorrichtung 40 weist ferner einen fünften Fluidkanal 49 zur Übertragung des Fluids zwischen dem ersten Fluidkanal 42 des ersten Anschlussstücks 41 und dem vierten Fluidkanal 48 des dritten Anschlussstücks 47 auf. Die Fluidtransfervorrichtung 40 ist dazu eingerichtet, in einem dritten Betriebsmodus betrieben zu werden, in welchem eine Kommunikationsverbindung zwischen der ersten Datenleitung 61 des ersten Anschlussstücks 41 und der dritten Datenleitung 65 des dritten Anschlussstücks 47 bereitgestellt wird. Wie in Fig. 6 dargestellt kann der Umschalter 71 beispielsweise als Umschalter mit einer weitere Ausgangsmöglichkeit ausgebildet sein.

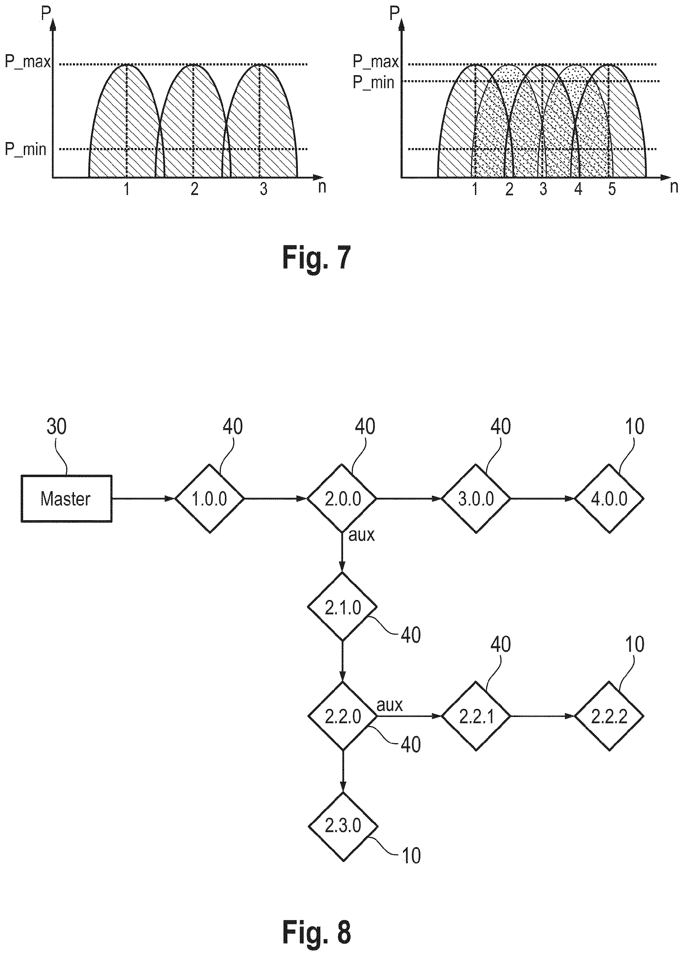

Fig. 7 zeigt beispielhafte Leistungsdiagramme, wobei die y-Achse die Leistung und die x-Achse eine Anzahl von Stufen einer Eingangsschaltung (siehe beispielsweise 72 in Fig. 5) zur Impedanzanpassung bezeichnet. Um eine Impedanz einer Ausgangsimpedanz von einer Quelle, hier beispielsweise der Hauptvorrichtung 30 (Fig. 1, Fig. 3) und einer Last, hier beispielsweise der Fluidtransfervorrichtung 40 oder des Medikationsbehälters 10, für eine vorteilhafte Signal- und/oder Energieübertragung anzupassen, wird eine Eingangsschaltung der Impedanzanpassung vorgeschlagen. Angenommen, die Last erfordert ein minimales Leistungslevel P_min, um zuverlässig arbeiten zu können, und ferner angenommen, dass ein maximales Leistungslevel P_max nicht überschritten werden sollte, um eine Beschädigung zu vermeiden, so ergibt sich ein Dynamikbereich (auch als Power Dynamic Range, PDR, bezeichnet) PDR=P_max - P_min. Bei einem geringen PDR kann eine feingliedrige Impedanzanpassung mit mehreren Stufen erforderlich sein (siehe Fig. 7, rechter Graph), wohingegen bei einem hohen PDR eine gröbere Anpassung (siehe Fig. 7, linker Graph) ausreichen kann.

In einer vorteilhaften Ausgestaltung weist die Elektronikeinrichtung 70 eine Eingangsschaltung 71 zur Impedanzanpassung auf, wobei die Eingangsschaltung dazu eingerichtet ist, abhängig von einer Position der Fluidtransfervorrichtung 40 in einer Verschaltung von mehreren Fluidtransfervorrichtungen, insbesondere einer Position der Fluidtransfervorrichtung in einer Reihenschaltung von mehreren Fluidtransfervorrichtungen, eine mit der Position assoziierte vordefinierte Impedanzanpassung einzustellen.

Fig. 8 zeigt eine schematische Darstellung mit Identifikation von Komponenten eines Fluidsystems. Die einzelnen Systemkomponenten könnten nacheinander durch ein sogenanntes "Sequential Call And Bypass", kurz SeCAB, identifiziert werden, um die Topologie des Fluidnetzwerks zu bestimmen. Die Systemkomponenten können sequenziell jeweils in den ersten Betriebsmodus versetzt und abgefragt werden und nachfolgend im zweiten Betriebsmodus per Bypass übergangen werden. Beispielsweise kann sich eine jeweilige Fluidtransfervorrichtung 40 nach dem Starten im ersten Betriebsmodus bei einer Hauptvorrichtung anmelden. Je nach Position im Netzwerk kann der jeweiligen Fluidtransfervorrichtung 40 oder dem Medikationsbehältern 10 eine ID zugewiesen werden. In Fig. 8 ist mit aux jeweils ein dritter Anschluss bzw. drittes Anschlussstück bezeichnet, wie beispielhaft im Kontext von Fig. 6 beschrieben.

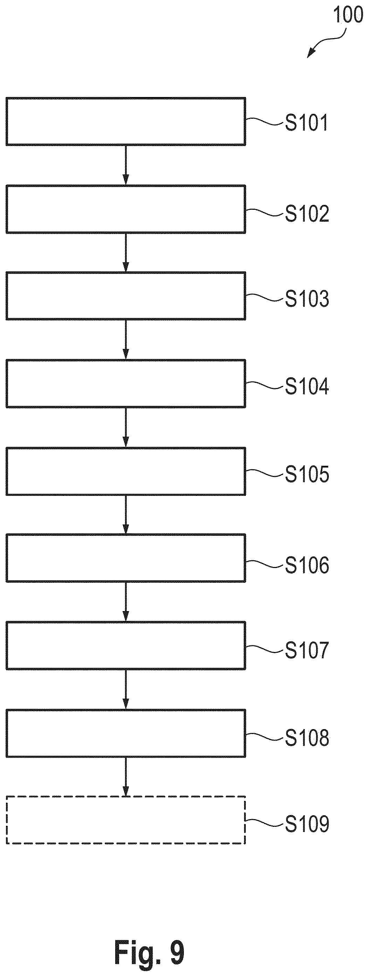

Fig. 9 zeigt ein Flussdiagramm eines Verfahrens 100 zur Versorgung eines Patienten mit einem medizinischen Fluid, insbesondere mit mindestens einem Medikament in flüssiger Form. In einem ersten Schritt S101 wird eine Hauptvorrichtung, insbesondere einer Infusionspumpe, bereitgestellt, wie im Rahmen der vorliegenden Offenbarung beschrieben. In einem zweiten Schritt S102 wird eine erste Fluidtransfervorrichtung bereitgestellt, wie im Rahmen der vorliegenden Offenbarung beschrieben. In einem dritten Schritt S103 wird eine zweite Fluidtransfervorrichtung bereitgestellt, wie im Rahmen der vorliegenden Offenbarung beschrieben. In einem vierten Schritt S104 wird ein erstes Anschlussstück der ersten Fluidtransfervorrichtung mit einem korrespondierenden Anschlussstück der Hauptvorrichtung gekoppelt. In einem fünften Schritt S105 wird ein erstes Anschlussstück der zweiten Fluidtransfervorrichtung mit einem korrespondierenden zweiten Anschlussstück der ersten Fluidtransfervorrichtung gekoppeltIn einem sechsten Schritt S106 wird die erste Fluidtransfervorrichtung zunächst in einem ersten Betriebsmodus, in welchem eine Kommunikationsverbindung zwischen der ersten Datenleitung des ersten Anschlussstücks und der Elektronikeinrichtung bereitgestellt wird, betrieben; und eine Kommunikation der Hauptvorrichtung mit der Elektronikeinheit der ersten Fluidtransfervorrichtung bereitgestellt. In einem siebten Schritt S107 wird die erste Fluidtransfervorrichtung nachfolgend in einem zweiten Betriebsmodus betrieben, in welchem eine Kommunikationsverbindung zwischen der ersten Datenleitung des ersten Anschlussstücks und der zweiten Datenleitung des zweiten Anschlussstücks bereitgestellt wird. In einem nachfolgenden achten Schritt S108 wird die zweite Fluidtransfervorrichtung in einem ersten Betriebsmodus betrieben, in welchem eine Kommunikationsverbindung zwischen der ersten Datenleitung des ersten Anschlussstücks der zweiten Fluidtransfervorrichtung und der Elektronikeinrichtung der zweiten Fluidtransfervorrichtung bereitgestellt wird; und eine Kommunikation der Hauptvorrichtung mit der Elektronikeinheit der zweiten Fluidtransfervorrichtung bereitgestellt.

Es versteht sich, dass die vorstehend genannten Schritte auch für weitere Fluidvorrichtungen und entsprechend auch für Medikationsbehälter als mögliche Endpunkte durchgeführt werden können. Es versteht sich, dass die Reihenfolge der Schritte nicht hierauf beschränkt ist und Schritte parallel ausgeführt werden können, soweit technisch sinnvoll.

Das System kann dazu eingerichtet sein, in einem optionalen Schritt S109, eine Netzwerktopologie der direkt oder indirekt an die Hauptvorrichtung angeschlossenen Fluidtransfervorrichtungen zu identifizieren durch sequenzielles Umschalten der ersten und zweiten Betriebsmodi der kaskadiert an die Hauptvorrichtung angeschlossenen Fluidtransfervorrichtungen.

Zusammenfassend können gemäß Aspekten der vorliegenden Offenbarung eine verbesserte Fluidtransfervorrichtung, ein System und Verfahren zur Versorgung eines Patienten mit einem medizinischen Fluid, insbesondere mit mindestens einem Medikament in flüssiger Form bereitgestellt werden. Insbesondere kann eine kostengünstige Herstellung zu ermöglicht werden, da es sich, wie vorstehend erläutert, bei Fluidtransfervorrichtungen um Einwegartikel handeln kann, wobei dennoch eine Topologie eines leitungsgebundenen medizinischen Fluidsystems äquitopologisch bestimmbar sein kann.

## Patentansprüche

1. Fluidtransfervorrichtung (40) zur Versorgung eines Patienten (2) mit einem medizinischen Fluid, insbesondere mit mindestens einem Medikament in flüssiger Form, mit den folgenden Merkmalen:
a. die Fluidtransfervorrichtung weist ein erstes Anschlussstück (41) mit einem ersten Fluidkanal (42) zur Übertragung des Fluids und einer ersten Datenleitung (61) zur Übertragung eines Kommunikationssignals auf,
b. die Fluidtransfervorrichtung weist ein zweites Anschlussstück (43) mit einem zweiten Fluidkanal (44) zur Übertragung des Fluids und einer zweiten Datenleitung (62) zur Übertragung eines Kommunikationssignals auf,
c. die Fluidtransfervorrichtung weist einen dritten Fluidkanal (45) zur Übertragung des Fluids zwischen dem ersten Fluidkanal (42) des ersten Anschlussstücks (41) und dem zweiten Fluidkanal (44) des zweiten Anschlussstücks (43) auf;
d. die Fluidtransfervorrichtung weist eine Elektronikeinrichtung (70) auf;
e. die Fluidtransfervorrichtung ist dazu eingerichtet, in einem ersten Betriebsmodus betrieben zu werden, in welchem eine Kommunikationsverbindung zwischen der ersten Datenleitung (61) des ersten Anschlussstücks (41) und der Elektronikeinrichtung (70) bereitgestellt wird; und
f. die Fluidtransfervorrichtung ist dazu eingerichtet, in einem zweiten Betriebsmodus betrieben zu werden, in welchem eine Kommunikationsverbindung zwischen der ersten Datenleitung (61) des ersten Anschlussstücks (41) und der zweiten Datenleitung (62) des zweiten Anschlussstücks (43) bereitgestellt wird.

2. Fluidtransfervorrichtung nach einem der vorhergehenden Ansprüche, mit dem folgenden zusätzlichen Merkmal:
a. das erste und/oder zweite Anschlussstück (41, 43) ist ein Luer-Konnektor, insbesondere ein Luer- Konnektor gemäß der Normenfamilie ISO 80369.

3. Fluidtransfervorrichtung nach einem der vorhergehenden Ansprüche, mit dem folgenden zusätzlichen Merkmal:
a. das erste und/oder zweite Anschlussstück (41, 43) ist zur kombinierten Übertragung des Fluids und zur induktiven Übertragung des Kommunikationssignals eingerichtet, insbesondere wobei zumindest ein Abschnitt der ersten Datenleitung (61) den ersten Fluidkanal umgibt (42) und/oder wobei zumindest ein Abschnitt der zweiten Datenleitung (62) den zweiten Fluidkanal (44) umgibt.

4. Fluidtransfervorrichtung nach einem der vorhergehenden Ansprüche, mit dem folgenden zusätzlichen Merkmal:
a. die Fluidtransfervorrichtung (40) ist dazu eingerichtet, in dem zweiten Betriebsmodus ein NFC-Signal drahtgebunden zwischen der ersten Datenleitung (61) des ersten Anschlussstücks (41) und der zweiten Datenleitung (62) des zweiten Anschlussstücks (43) weiterzuleiten.

5. Fluidtransfervorrichtung nach einem der vorhergehenden Ansprüche, mit dem folgenden zusätzlichen Merkmal:
a. die Fluidtransfervorrichtung (40) ist dazu eingerichtet, in dem ersten Betriebsmodus die zweite Datenleitung (62) des zweiten Anschlussstücks (43) von der ersten Datenleitung (61) des ersten Anschlussstücks (41) zu trennen.

6. Fluidtransfervorrichtung nach einem der vorhergehenden Ansprüche, mit dem folgenden zusätzlichen Merkmal:
a. die Fluidtransfervorrichtung () ist dazu eingerichtet, in dem zweiten Betriebsmodus die erste Datenleitung (61) des ersten Anschlussstücks (41) von der Elektronikeinrichtung (70) zu trennen.

7. Fluidtransfervorrichtung nach den Ansprüchen 5 und 6, mit den folgenden zusätzlichen Merkmalen:
a. wobei die erste Datenleitung (61) als differentielle erste Datenleitung ausgebildet ist,
b. wobei die zweite Datenleitung (62) als differentielle zweite Datenleitung ausgebildet ist; und
c. wobei die Fluidtransfervorrichtung einen Doppelpol-Umschalter (71), insbesondere einen Halbleiter DPDT-Schalter, zur Umschaltung zwischen dem ersten Betriebsmodus und dem zweiten Betriebsmodus aufweist.

8. Fluidtransfervorrichtung nach einem der vorhergehenden Ansprüche, mit dem folgenden zusätzlichen Merkmal:
a. die Fluidtransfervorrichtung (40) ist dazu eingerichtet, in dem ersten Betriebsmodus zu starten; und
b. die Fluidtransfervorrichtung (40) ist dazu eingerichtet, selbsttätig, insbesondere nach einer vorgegebenen Zeitdauer, aus dem zweiten Betriebsmodus in den ersten Betriebsmodus zu wechseln.

9. Fluidtransfervorrichtung nach einem der vorhergehenden Ansprüche, mit dem folgenden zusätzlichen Merkmal:
a. die Fluidtransfervorrichtung () ist dazu eingerichtet, als Reaktion auf ein über die erste Datenleitung (61) des ersten Anschlussstücks (41) erhaltenes Steuersignal von dem ersten Betriebsmodus in den zweiten Betriebsmodus zu wechseln.

10. Fluidtransfervorrichtung nach einem der vorhergehenden Ansprüche, mit dem folgenden zusätzlichen Merkmal:
a. die Fluidtransfervorrichtung (40) ist dazu eingerichtet, induktiv über die Datenleitung (61) des ersten Anschlussstücks (41) mit Energie versorgt zu werden.

11. Fluidtransfervorrichtung nach einem der vorhergehenden Ansprüche, mit dem folgenden zusätzlichen Merkmal:
a. die Elektronikeinrichtung (70) weist eine Eingangsschaltung (71) zur Impedanzanpassung auf, wobei die Eingangsschaltung dazu eingerichtet ist, abhängig von einer Position der Fluidtransfervorrichtung (40) in einer Verschaltung von mehreren Fluidtransfervorrichtung en, insbesondere einer Position der Fluidtransfervorrichtung in einer Reihenschaltung von mehreren Fluidtransfervorrichtung en, eine mit der Position assoziierte vordefinierte Impedanzanpassung einzustellen.

12. Fluidtransfervorrichtung nach einem der vorhergehenden Ansprüche, mit den folgenden zusätzlichen Merkmalen:
a. die Fluidtransfervorrichtung weist ein drittes Anschlussstück (47) mit einem vierten Fluidkanal (48) zur Übertragung des Fluids und einer dritten Datenleitung (65) zur Übertragung eines Kommunikationssignals auf;
b. die Fluidtransfervorrichtung weist einen fünften Fluidkanal (49) zur Übertragung des Fluids zwischen dem ersten Fluidkanal (45) des ersten Anschlussstücks (41) und dem vierten Fluidkanal (48) des dritten Anschlussstücks (47) auf; und
c. die Fluidtransfervorrichtung ist dazu eingerichtet, in einem dritten Betriebsmodus betrieben zu werden, in welchem eine Kommunikationsverbindung zwischen der ersten Datenleitung (61) des ersten Anschlussstücks (41) und der dritten Datenleitung (65) des dritten Anschlussstücks (47) bereitgestellt wird.

13. System (1) zur Versorgung eines Patienten mit einem medizinischen Fluid, insbesondere mit mindestens einem Medikament in flüssiger Form, mit den folgenden Merkmalen:
a. einer Hauptvorrichtung (30), insbesondere einer Infusionspumpe;
b einer ersten Fluidtransfervorrichtung (40) nach einem der vorhergehenden Ansprüche;
c. einer zweiten Fluidtransfervorrichtung (40) nach einem der vorhergehenden Ansprüche;
d. wobei ein erstes Anschlussstück (41) der ersten Fluidtransfervorrichtung (40) dazu eingerichtet ist, mit einem korrespondierenden Anschlussstück der Hauptvorrichtung (30) gekoppelt zu werden;
e. wobei ein erstes Anschlussstück (41) der zweiten Fluidtransfervorrichtung (40) dazu eingerichtet ist, mit einem korrespondierenden zweiten Anschlussstück (43) der ersten Fluidtransfervorrichtung gekoppelt zu werden;
f. wobei das System (1) dazu eingerichtet ist, die erste Fluidtransfervorrichtung (40) zunächst in einem ersten Betriebsmodus zu betreiben, in welchem eine Kommunikationsverbindung zwischen der ersten Datenleitung (61) des ersten Anschlussstücks (41) und der Elektronikeinrichtung (70) bereitgestellt wird, um eine Kommunikation der Hauptvorrichtung (30) mit der Elektronikeinheit (70) der ersten Fluidtransfervorrichtung (40) bereitzustellen; und
g. wobei das System (1) dazu eingerichtet ist,
die erste Fluidtransfervorrichtung (40) nachfolgend in einem zweiten Betriebsmodus zu betreiben, in welchem eine Kommunikationsverbindung zwischen der ersten Datenleitung (61) des ersten Anschlussstücks (41) und der zweiten Datenleitung (62) des zweiten Anschlussstücks (43) bereitgestellt wird; und
die zweite Fluidtransfervorrichtung (40) in einem ersten Betriebsmodus zu betreiben, in welchem eine Kommunikationsverbindung zwischen der ersten Datenleitung (61) des ersten Anschlussstücks (41) der zweiten Fluidtransfervorrichtung und der Elektronikeinrichtung (70) der zweiten Fluidtransfervorrichtung bereitgestellt wird, um eine Kommunikation der Hauptvorrichtung (30) mit der Elektronikeinheit (70) der zweiten Fluidtransfervorrichtung (40) bereitzustellen.

14. System nach Anspruch 13, mit dem folgenden zusätzlichen Merkmal:
a. das System ist dazu eingerichtet, eine Netzwerktopologie der direkt oder indirekt an die Hauptvorrichtung angeschlossenen Fluidtransfervorrichtungen zu identifizieren durch sequenzielles Umschalten der ersten und zweiten Betriebsmodi der kaskadiert an die Hauptvorrichtung angeschlossenen Fluidtransfervorrichtungen.

15. Verfahren (100) zur Bereitstellung eines System zur Versorgung eines Patienten mit einem medizinischen Fluid, insbesondere mit mindestens einem Medikament in flüssiger Form, mit den folgenden Merkmalen:
a. Bereitstellen einer Hauptvorrichtung, insbesondere einer Infusionspumpe (S101);
b Bereitstellen einer ersten Fluidtransfervorrichtung nach einem der vorhergehenden Ansprüche (S102);
c. Bereitstellen einer zweiten Fluidtransfervorrichtung nach einem der vorhergehenden Ansprüche (S103);
d. Koppeln eines ersten Anschlussstücks der ersten Fluidtransfervorrichtung mit einem korrespondierenden Anschlussstück der Hauptvorrichtung (S104);
e. Koppeln eines ersten Anschlussstücks der zweiten Fluidtransfervorrichtung mit einem korrespondierenden zweiten Anschlussstück der ersten Fluidtransfervorrichtung (S105);
f. Betreiben der ersten Fluidtransfervorrichtung zunächst in einem ersten Betriebsmodus, in welchem eine Kommunikationsverbindung zwischen der ersten Datenleitung des ersten Anschlussstücks und der Elektronikeinrichtung bereitgestellt wird; und Bereitstellen einer Kommunikation der Hauptvorrichtung mit der Elektronikeinheit der ersten Fluidtransfervorrichtung (S106);
g. Betreiben der ersten Fluidtransfervorrichtung nachfolgend in einem zweiten Betriebsmodus, in welchem eine Kommunikationsverbindung zwischen der ersten Datenleitung des ersten Anschlussstücks und der zweiten Datenleitung des zweiten Anschlussstücks bereitgestellt wird (S107); und
h. Betreiben der zweiten Fluidtransfervorrichtung in einem ersten Betriebsmodus, in welchem eine Kommunikationsverbindung zwischen der ersten Datenleitung des ersten Anschlussstücks der zweiten Fluidtransfervorrichtung und der Elektronikeinrichtung der zweiten Fluidtransfervorrichtung bereitgestellt wird; und Bereitstellen einer Kommunikation der Hauptvorrichtung mit der Elektronikeinheit der zweiten Fluidtransfervorrichtung (S108).
